# EUROPÄISCHE PATENTSCHRIFT

(11) **EP 2 442 836 B1**
(45) Veröffentlichungstag und Bekanntmachung des Hinweises auf die Patenterteilung: **05.08.2015**
(21) Anmeldenummer: 10744684.1
(22) Anmeldetag: 21.06.2010
(51) Int. Cl.: A61L 27/04, A61L 27/58, A61L 31/02, A61L 31/14

(54) **IMPLANTAT MIT EINEM VOM KÖRPER RESORBIERBAREN METALLISCHEN WERKSTOFF**
IMPLANT MADE OF A METALLIC MATERIAL WHICH CAN BE RESORBED BY THE BODY
IMPLANT CONSTITUÉ D'UN MATÉRIAU MÉTALLIQUE POUVANT ÊTRE RÉSORBÉ PAR L'ORGANISME

(30) Priorität: 19.06.2009 DE 102009025511
(43) Veröffentlichungstag der Anmeldung: 25.04.2012
(73) Patentinhaber: QUALIMED Innovative Medizinprodukte Gesellschaft mit beschränkter Haftung, 21423 Winsen/Luhe (DE)
(72) Erfinder: GÜLCHER, Manfred, 46348 Raesfeld-Erle (DE)
(74) Vertreter: Schneiders, Josef
(86) Internationale Anmeldenummer: PCT/EP2010/003723
(87) Internationale Veröffentlichungsnummer: WO 2010/145842

(56) Entgegenhaltungen:
- EP-A2- 1 270 023
- CN-A- 101 283 922
- CN-B- 101 085 377
- DE-A1- 1 953 241
- DE-U1- 20 202 591

## Beschreibung

Die Erfindung betrifft ein Implantat mit einem vom Körper resorbierbaren metallischen Werkstoff in Form einer Magnesiumlegierung.

Im Körper eines Patienten resorbierbare Implantate sind grundsätzlich seit längerem bekannt. Zum einen wäre auf Implantate aus biokompatiblen und bioresorbierbaren Kunststoffen hinzuweisen, etwa aus Polylactiden und Polyglykoliden, die beispielsweise bei Stentbeschichtungen eingesetzt werden und sich unter Abgabe eines darin eingeschlossenen Wirkstoffes über einen kürzeren oder längeren Zeitraum auflösen. Es wurde auch vorgeschlagen, Implantate selbst aus solchen Kunststoffe herzustellen, beispielsweise Stents. Resorbierbare Implantate aus metallischen Werkstoffe wurden verschiedentlich vorgeschlagen, haben bislang jedoch keine verbreitete Anwendung gefunden. So beschreibt beispielsweise die EP 0 923 389 einen Reineisenstent, der sich in vivo über einen Zeitraum von vier bis 12 Wochen praktisch vollständig abbaut. Die beim biologischen Abbau der Stentstruktur auftretenden Abbaumuster, die vielfach die Form von filigranen Spitzen annehmen, sind allerdings geeignet, im Nachhinein Gefäßverletzungen zu verursachen.

Gemäß EP 1 270 023 A2 können medizinische Implantate aus einer Reihe von Magnesiumlegierungen gefertigt werden, die u.a. Lithium, Aluminium, Seltenerdmetalle, Mangan, Zink oder andere Metalle enthalten können. Konkret sind Legierungen aus 8,5 bis 9,5 % Aluminium, 0,15 bis 0,4 % Mangan, 0,45 bis 0,9 % Zink und Rest Magnesium wie auch aus 4,5 bis 5,3 % Aluminium, 0,28 bis 0,5 % Mangan und Rest Magnesium beschrieben. Durch den Aluminiumgehalt wird die Zugfestigkeit und Härte der Legierung bestimmt, der Mangangehalt bestimmt die Korrosionbeständigkeit.

Aus der DE-OS 1 953 241 ist eine Magnesiumlegierung für die Knochenchirurgie bekannt, die neben Mangan und Seltenerdmetall noch Cadmium und Calcium oder Aluminium enthält. Die CN 1010 85 377 B beschreibt Magnesiumlegierungen für Stents, die neben Mangan und Seltenerdmetallen noch Yttrium, Aluminium, Calcium, Zink, Antimon und Zirkonium enthalten.

Magnesium ist ein für den menschlichen Körper essentielles Element und physiologisch völlig unbedenklich. Die Verwendung von Aluminium in Implantaten ist allerdings umstritten. Aluminiumionen können gesundheitliche Schäden verursachen und Demenz und Gedächtnisverlust führen. Weiterhin gilt Aluminium als Risikofaktor für die Alzheimerkrankheit. Insoweit stellt die Verwendung von Aluminium in bioresorbierbaren Stents aus denen zwingend Aluminiumionen abgegeben werden, ein unerwünschtes Risiko dar.

Seltenerdmetalle können zwar in Ionenform in höherer Konzentration gerinnungshemmend wirken, gelten jedoch in geringen Mengen als unbedenklich. Eisen-, Zink- und Manganionen gelten physiologisch als unbedenklich und sind essentielle Spurenelemente.

Bei der Fertigung resorbierbarer magnesiumhaltiger Stents sind auch die chemischen und mechanischen Eigenschaften von Magnesium und seinen Legierungen zu beachten. Chemisch gesehen ist Magnesium leicht korrodierbar und wird ohne weitere Schutzmaßnahme im menschlichen Gefäßsystem innerhalb kurzer Zeit vollständig aufgelöst. Die Auflösungszeiten können über die Legierungsmetalle beeinflusst werden.

Ein chemisches Problem bei der Verwendung von Magnesium in Stents ist die bei Resorption auftretende Bildung von Wasserstoffgas, die der praktischen Anwendung von Magnesiumstents in der Kardiologie prinzipiell entgegensteht. Wasserstoffgas in Bläschenform kann zu Embolien und damit zur Schädigung des Patienten führen.

Der schnellen Resorption kann man durch Zulegieren anderer Metalle entgegenwirken und damit zu einer gesteuerten Auflösung gelangen. Gute Ergebnisse werden mit Manganzusätzen bis zu 0,5 Gew.-% erzielt. Dennoch bleiben die Standzeiten solcher Legierungen bei Implantaten, die eine Stützfunktion, beispielsweise bei der Stabilisierung von Frakturen und bei der Erweiterung von koronaren Blutgefäßen ausüben sollen, häufig deutlich zu kurz.

Damit die Implantate ihre Stützfunktion, insbesondere auch bei der Fertigung medizinischer Nägel, voll gerecht werden, benötigen sie eine Dauerfestigkeit in der Anfangsphase, die über mehrere Wochen bis Monate reichen sollte. Eine hinreichende Festigkeit wird von Magnesium allein nicht erreicht. Magnesium-Aluminium-Legierungen haben diese Festigkeit, in Gegenwart von Manganzusätzen auch zumeist eine hinreichende Dauerfestigkeit.

Benötigt wird weiterhin eine gute Verformbarkeit bzw. Duktilität der Legierung, die insbesondere bei der Fertigung der Implantierung von Stents eine Rolle spielt. Stents werden nach der Fertigung aus einem Rohr auf einen Dillatationsballon gekrimpt und dann bei der Implantierung im Gefäß mechanisch über den alten Rohrdurchmesser hinaus erweitert. Diese doppelte mechanische Beanspruchung verlangt eine gute Kaltverformbarkeit des Materials, so dass der magnesiumhaltige Stent nach der Implantierung ohne nennenswerte strukturelle Schäden im Blutgefäß verankert ist. Strukturelle Schäden führen zu Einsatzpunkten für die Korrosion und im Ergebnis zu einer vorzeitigen Auflösung bzw. Schwächung des Konstrukts. Magnesiumlegierungen mit weniger als 10 Gew.-% Aluminiumanteil haben in der Regel sowohl die notwendige Festigkeit wie Verformbarkeit.

Insgesamt besteht deshalb ein Bedarf an einem Material, mit dem Implantate gefertigt werden können, die die oben geschilderten Nachteile der bekannten Implantate aus resorbierbaren Metalllegierungen nicht aufweisen.

Aufgabe der Erfindung ist deshalb die Bereitstellung eines Implantats aus einer Magnesiumlegierung, die im menschlichen oder tierischen Körper resorbierbar ist, frei von Aluminium ist, bei der Resorption allenfalls geringe Menge Wasserstoff entwickelt und über die benötigte Standfestigkeit verfügt.

Überraschend wurde gefunden, dass diese Aufgabe mit einem Implantat der eingangs genannten Art gelöst wird, bei dem der metallische Werkstoff eine Magnesiumlegierung ist, die aus 96 bis 97,9 Gew.-% Magnesium, 1,6 bis 2 Gew.-% Mangan und 0,5 bis 2 Gew.-% wenigstens eines Metalls der Seltenerdgruppe besteht.

Als Metalle der Seltenerdgruppe werden Scandium, Yttrium und Lanthan sowie die auf Lanthan folgenden und als Lanthanide bezeichneten Elemente des Periodensystems der Elemente verstanden. Im Einzelnen zu nennen sind hier die Elemente Cer, Praeseodym, Neodym, Promethium, Samarium, Europium, Gadolinium, Terbium, Dysprosium, Holmium, Erbium, Thulium, Ytterbium und Lutetium. Bevorzugt ist Cer.

Der metallische Werkstoff der erfindungsgemäßen Implantate kann aus Massivstrukturen, Gitterstrukturen, Draht- oder Gewebestrukturen wie auch aus Metallschaum oder porösem Metall bestehen. Der resorbierbare metallische Werkstoff kann mit anderen nicht-organischen Werkstoffen kombiniert werden, beispielsweise mit Keramikwerkstoffen oder mit Bioglas.

Bei den erfindungsgemäßen Implantaten handelt es sich beispielsweise um vaskuläre oder nicht vaskuläre Stents.

Schwamm- und poröse Strukturen haben den Vorteil einer erhöhten Resorptionsgeschwindigkeit, wobei die vorhandenen Poren das Einwachsen von körpereigenem Gewebe ermöglichen. Dies erlaubt die Verwendung von Magnesiumschwammkörpern als Platzhalter beispielsweise bei der Behandlung von Frakturen und der Augmentierung von Knochen- und anderem Körpergewebe.

Es versteht sich, dass Struktur und Festigkeit des metallischen Werkstoffes für die erfindungsgemäßen Implantate an den Einsatzort und Einsatzzweck angepasst sind, beispielsweise hinsichtlich ihrer mechanischen Eigenschaften und/oder als Drug-Delivery-Systeme.

Insbesondere beim Einsatz als Knochenersatz können die resorbierbaren metallischen Werkstoffe durch geeignete Bearbeitung, etwa durch Lasersintern nach CAD-Daten, an den Einsatzort genau angepasst und dort passgenau eingesetzt werden. Dies erlaubt die Nachmodellierung von beispielsweise Wangenknochen, Teilen der Schädeldecke und dergleichen, die nach Unfällen oder operativen Behandlungen ersetzt werden müssen.

Die erfindungsgemäßen Implantate können ganz oder teilweise aus einem solchen resorbierbaren metallischen Werkstoff bestehen. Neben dem erfindungsgemäßen resorbierbaren metallischen Werkstoff können dies resorbierbare oder nicht-resorbierbare andere metallische oder nicht-metallische Werkstoffe sein. Insbesondere können diese weiteren Bestandteile Kunststoffe sein, die aus einem resorbierbaren Material bestehen, etwa einem Polylactid oder Polyglycolid. Solche resorbierbaren Kunststoffe werden häufig als Beschichtungen von Stents eingesetzt und werden von der Firma Boehringer Ingelheim unter der Bezeichnung Resomer^{®} vertrieben. Desgleichen können Chitin- und Chitosan-Biopolymere zur Beschichtung verwandt werden. Beschichtungen dienen häufig als Träger von Medikamenten, die daraus langsam an die Umgebung abgegeben werden.

Die resorbierbaren metallischen Werkstoffe können auch in Form eines Pulvers in eine aushärtende Paste, beispielsweise in Abmischung mit einem Resomer^{®}, Chitin- oder Chitosan-Biopolymer, mit Hyaluronsäure, Alginat oder Chonditrinsulfat, eingebracht werden und als solche zur Augmentierung, aber auch zur Nahtabdichtung im Darmbereich eingesetzt werden. Das ausgehärtete Implantat erfüllt am Einsatzort eine Stütz- und Schutzfunktion, erlaubt aber gleichzeitig die Abheilung des Defekts bzw. der Naht, bevor es vom Körper resorbiert wird.

Erfindungsgemäß besteht die erfindungsgemäße resorbierbare Magnesiumlegierung aus 96 bis 97,9 Gew.-% Magnesium, 1,6 bis 2 Gew.-% Mangan und 0,5 bis 2 Gew.-% Seltenerdmetall. Dabei wird vorzugsweise Neodym oder Cer als Seltenerdmetall verwandt. Besonders bewährt hat sich eine Zusammensetzung aus 97,45 Gew.-% Magnesium, 1,8 Gew.-% Mangan und 0,75 Gew.-% Cer. Eine solche Legierung hat sich insbesondere im Fahrzeugbau bei Halterungen, Stützen, Sitz-, Fenster- oder Türrahmen, Gehäusen, Trägern und Kleinteilen bewährt und ist in DE 202 02 591 U1 beschrieben. Medizinische Anwendungen dieser Legierung sind bislang nicht bekannt geworden.

## Patentansprüche

1. Implantat mit einem vom Körper resorbierbaren metallischen Werkstoff, **dadurch gekennzeichnet, dass** der metallische Werkstoff eine Magnesiumlegierung ist, die aus 96 bis 97,9 Gew.-% Magnesium, 1,6 bis 2 Gew.-% Mangan und 0,5 bis 2 Gew.-% Seltenerdmetall besteht.

2. Implantat nach Anspruch 1, **dadurch gekennzeichnet, dass** das Seltenerdmetall Neodym oder Cer ist.

3. Implantat nach Anspruch 2, **dadurch gekennzeichnet, dass** das Implantat aus 97,45 Gew.-% Magnesium, 1,8 Gew.-% Mangan und 0,75 Gew.-% Cer oder Neodym besteht.

4. Implantat nach einem der vorstehenden Ansprüche in Form eines Stents, Nagels, einer Schraube oder einer Platte für die Fixierung von Frakturen.

5. Implantat nach einem der Ansprüche 1 bis 3 in Form eines vaskulären Implantats.

6. Implantat nach Anspruch 5 in Form eines Stents.

7. Implantat nach Anspruch 6 in Form eines mit einem resorbierbaren Biopolymeren beschichteten Stents.

8. Stent nach Anspruch 7, **dadurch gekennzeichnet, dass** er einen proliferationshemmenden Wirkstoff enthält.

9. Stent nach Anspruch 8, **dadurch gekennzeichnet, dass** der resorbierbare Kunststoff Rapamycin oder Paclitaxel enthält.

10. Verwendung einer Magnesiumlegierung, die aus 96 bis 97,9 Gew.-% Magnesium, 1,6 bis 2 Gew.-% Mangan und 0,5 bis 2 Gew.-% Seltenerdmetall besteht, zur Herstellung von medizinischen Implantaten.

11. Verwendung nach Anspruch 10, **dadurch gekennzeichnet, dass** das Seltenerdmetall Cer oder Neodym ist.

12. Verwendung nach einem der Ansprüche 10 oder 11, **dadurch gekennzeichnet, dass** die Magnesiumlegierung aus 97,45 Gew.-% Magnesium, 1,8 Gew.-% Mangan und 0,75 Gew.-% Cer oder Neodym besteht.

## Claims

1. Implant consisting of a material resorbable by the body, **characterized in that** the metallic material is a magnesium alloy consisting of 96 to 97.9 % w/w of magnesium, 1.6 to 2 % w/w of manganese and 0.5 to 2 % w/w of rare earth metal.

2. Implant according to claim 1, **characterized in that** the rare earth metal is neodymium or cerium.

3. Implant according to claim 2, **characterized in that** the implant consists of 97.45 % w/w of magnesium, 1.8 % w/w of manganese and 0.75 % w/w of cerium or neodymium.

4. Implant according to any one of the above claims in the form of a stent, nail, screw or plate for the fixation of fractures.

5. Implant according to any one of claims 1 to 3 in the form of a vascular implant.

6. Implant according to claim 5 in the form of a stent.

7. Implant according to claim 6 in the form of a stent coated with a resorbable biopolymer.

8. Stent according to claim 7, **characterized in that** said stent contains a proliferation-inhibiting active agent.

9. Stent according to claim 8, **characterized in that** the resorbable plastic material contains rapamycin or paclitaxel.

10. Use of a magnesium alloy consisting of 96 to 97.9 % w/w of magnesium, 1.6 to 2 % w/w of manganese and 0.5 to 2 % w/w of rare earth metal for the manufacture of medical implants.

11. Use according to claim 10, **characterized in that** the rare earth metal is cerium or neodymium.

12. Use according to any one of claims 10 or 11, **characterized in that** the magnesium alloy consists of 97.45% w/w of magnesium, 1.8% w/w of manganese and 0.75 % w/w of cerium or neodymium.

## Revendications

1. Implant comprenant un matériau métallique pouvant être résorbé par l'organisme,
**caractérisé en ce que** le matériau métallique est un alliage de magnésium constitué de 96 à 97,9 % en poids de magnésium, 1,6 à 2 % en poids de manganèse et 0,5 à 2 % en poids de métal des terres rares.

2. Implant selon la revendication 1, **caractérisé en ce que** le métal des terres rares est du néodyme ou du cérium.

3. Implant selon la revendication 2, **caractérisé en ce que** l'implant est constitué de 97,45 % en poids de magnésium, de 1,8 % en poids de manganèse et de 0,75 % en poids de cérium ou néodyme.

4. Implant selon l'une des revendications précédentes sous la forme d'un stent, d'un clou, d'une vis ou d'une plaque pour fixer des fractures.

5. Implant selon l'une quelconque des revendications 1 à 3 sous la forme d'un implant vasculaire.

6. Implant selon la revendication 5 sous la forme d'un stent.

7. Implant selon la revendication 6 sous la forme d'un stent revêtu d'un bio-polymère pouvant être résorbé.

8. Stent selon la revendication 7, **caractérisé en ce qu'**il comprend un agent anti-prolifératif.

9. Stent selon la revendication 8, **caractérisé en ce que** la matière synthétique pouvant être résorbée contient de la rapamycine ou du paclitaxel.

10. Utilisation d'un alliage de magnésium constitué de 96 à 97,9 % en poids de magnésium, 1,6 à 2 % en poids de manganèse et 0,5 à 2 % en poids de métal des terres rares pour la fabrication d'implants médicaux.

11. Utilisation selon la revendication 10, **caractérisé en ce que** le métal des terres rares est du néodyme ou du cérium.

12. Utilisation selon la revendication 10 ou 11, **caractérisé en ce que** l'alliage de magnésium est constitué de 97,45 % en poids de magnésium, de 1,8 % en poids de manganèse et de 0,75 % en poids de cérium ou néodyme.
